# EUROPEAN PATENT SPECIFICATION

(11) **EP 2 121 564 B1**
(45) Date of publication and mention of the grant of the patent: **06.11.2013**
(21) Application number: 08708009.9
(22) Date of filing: 21.01.2008
(51) Int. Cl.: C07C 68/06, C07C 69/96, C07C 29/128

(54) **PROCESS FOR THE PREPARATION OF DIARYL CARBONATE**
VERFAHREN ZUR HERSTELLUNG VON DIARYLCARBONAT
PROCÉDÉ DE PRÉPARATION DE CARBONATE DE DIARYL

(30) Priority: 23.01.2007 EP 07100969
(43) Date of publication of application: 25.11.2009
(73) Proprietor: Shell Internationale Research Maatschappij B.V., 2596 HR Den Haag (NL)
(72) Inventor: NISBET, Timothy Michael, NL-1031 CM Amsterdam (NL); VAPORCIYAN, Garo Garbis, Houston, Texas 77079 (US); VROUWENVELDER, Cornelis Leonardo Maria, NL-1031 CM Amsterdam (NL); WOOD, Paul, NL-1031 CM Amsterdam (NL)
(74) Representative: Matthezing, Robert Maarten
(86) International application number: PCT/EP2008/050605
(87) International publication number: WO 2008/090107

(56) References cited:
- WO-A-03/000641
- JP-A- 9 040 616

## Description

- The present invention relates to a process for the preparation of diaryl carbonate by reaction of an aromatic alcohol with a dialkyl carbonate.

Diaryl carbonates are important starting materials for the production of aromatic polycarbonates. Polycarbonates can be manufactured by polymerisation of a diaryl carbonate with an aromatic dihydroxy compound. In this reaction an aromatic alcohol is produced as by-product. This by-product can be usefully recycled to the production of the diaryl carbonate from dialkyl carbonate with the aromatic alcohol.

The production of polycarbonate by the polymerisation of diaryl carbonate with an aromatic dihydroxy compound is known from US-A 5,747,609. This document also describes the production of diaryl carbonate from dialkyl carbonate with an aromatic alcohol. In this reaction a transesterification of dialkyl carbonate takes place whereby overall the diaryl carbonate is produced and as by-product alkanol is obtained. The dialkyl carbonate can be produced from an alkylene carbonate and an alkanol. This reaction yields as useful products not only the dialkyl carbonate that can be used for the preparation of diaryl carbonate, but also a useful alkanediol. For this reaction one may use the alkanol that has been liberated in the transesterification of dialkyl carbonate and aromatic alcohol.

A process wherein alkanol that has been liberated in the transesterification of dialkyl carbonate and aromatic alcohol is used in the preparation of dialkyl carbonate is known from Japanese Patent Application Publication No. JP-A 09040616. This document describes the reaction of propylene carbonate with methanol to yield a mixture of dimethyl carbonate and methanol on the one hand and 1,2-propanediol as product on the other hand. The mixture of dimethyl carbonate and methanol is subjected to extractive distillation. The document further describes that phenol and dimethyl carbonate are reacted in a transesterification zone to yield a diphenyl carbonate stream and a stream comprising unreacted dimethyl carbonate and methanol. Both the diphenyl carbonate stream and the stream comprising dimethyl carbonate and methanol contain phenol. The diphenyl carbonate stream is therefore subjected to distillation to yield purified diphenyl carbonate and phenol, which phenol is recycled to the transesterification zone. The phenol-contaminated stream comprising dimethyl carbonate and methanol is subjected to separate distillation to obtain a purified stream of dimethyl carbonate and methanol, and a separate stream of phenol. Subsequently, the purified stream of dimethyl carbonate and methanol is fed to the same extractive distillation as where the first mixture of dimethyl carbonate and methanol are being treated. In the extractive distillation an extractant is being used. Examples of extractants are alkylene carbonates, e.g. those that are being used as starting material for the preparation of dimethyl carbonate.

Such a process for the production of dialkyl carbonate from the reaction of an alkylene carbonate and an alkanol, in which a dialkyl carbonate/alkanol mixture is subjected to an extractive distillation with the same alkylene carbonate as extractant is also known from WO-A 03/006418.

One of the issues in the integration of the process for the production of diaryl carbonate and the process for the production of dialkyl carbonate and alkandiol (alkylene glycol) is the potential contamination of the alkylene glycol with the aromatic alcohol, such as phenol. Such contamination is highly undesirable. Moreover, the purification of phenol-contaminated alkylene glycol products is very cumbersome. The Applicants of JP 09040616 have solved this problem by adding a dedicated distillation column to purify the phenol-contaminated stream of dimethyl carbonate and methanol. Any phenol is thereby removed from this stream and the purified stream can then be recycled to the extractive distillation zone to separate methanol from dimethyl carbonate. The methanol can subsequently be recycled to the reaction of methanol and propylene carbonate without running the,risk of either contamination of the eventual propylene glycol product or build-up of phenol in the extractant of the extractive distillation.

The avoidance of such risk therefore comes at the expense of an additional distillation column.

The present invention provides a process in which the use of such an expensive distillation column is no longer needed.

Accordingly, the present invention provides a process for the preparation of diaryl carbonate by reaction of an aromatic alcohol with a dialkyl carbonate, which dialkyl carbonate has been prepared by the reaction of an alkanol and an alkylene carbonate, which process comprises the following steps:
(a) passing an aromatic alcohol and a dialkyl carbonate into a first transesterification zone to obtain a first product stream containing diaryl carbonate, alkanol, unconverted dialkyl carbonate and unconverted aromatic alcohol;
(b) separating the first product stream into a diaryl carbonate-rich product stream, an aromatic alcohol-rich recycle stream and a second recycle stream comprising alkanol, dialkyl carbonate and aromatic alcohol;
(c) feeding alkanol and alkylene carbonate into a second transesterification zone to obtain a second product stream comprising alkanediol and unconverted alkanol and unconverted dialkyl carbonate;
(d) separating alkanediol from the second product stream to yield an alkanediol product stream and a mixture of dialkyl carbonate and unconverted alkanol;
(e) subjecting the mixture of dialkyl carbonate and unconverted alkanol and the second recycle stream comprising alkanol, dialkyl carbonate and aromatic alcohol to the same distillation to obtain an alkanol stream as the lower-boiling fraction and a contaminated stream comprising dialkyl carbonate and aromatic alcohol as the higher-boiling fraction;
(f) recycling the alkanol stream of step e) to the second transesterification zone; and
(g) passing the contaminated stream comprising dialkyl carbonate and aromatic alcohol, and the aromatic alcohol-rich recycle stream to the first transesterification zone.

This process allows that an aromatic alcohol-contaminated stream of dialkyl carbonate and alkanol can be subjected to the same distillation as the mixture of dialkyl carbonate and alkanol obtained as the product of the second transesterification zone. Since the distillation is conducted such that the alkanol is obtained over the top of the column and the dialkyl carbonate with any aromatic alcohol is obtained at the bottom of the column, and since the contaminated dialkyl carbonate thus obtained is passed to the first transesterification zone, there is no risk that the second transesterification zone is contaminated with aromatic alcohol with undesired disastrous effects on the purity of the alkanediol product. Moreover, all aromatic alcohol is recycled to the first transesterification zone without the need for an additional separate distillation.

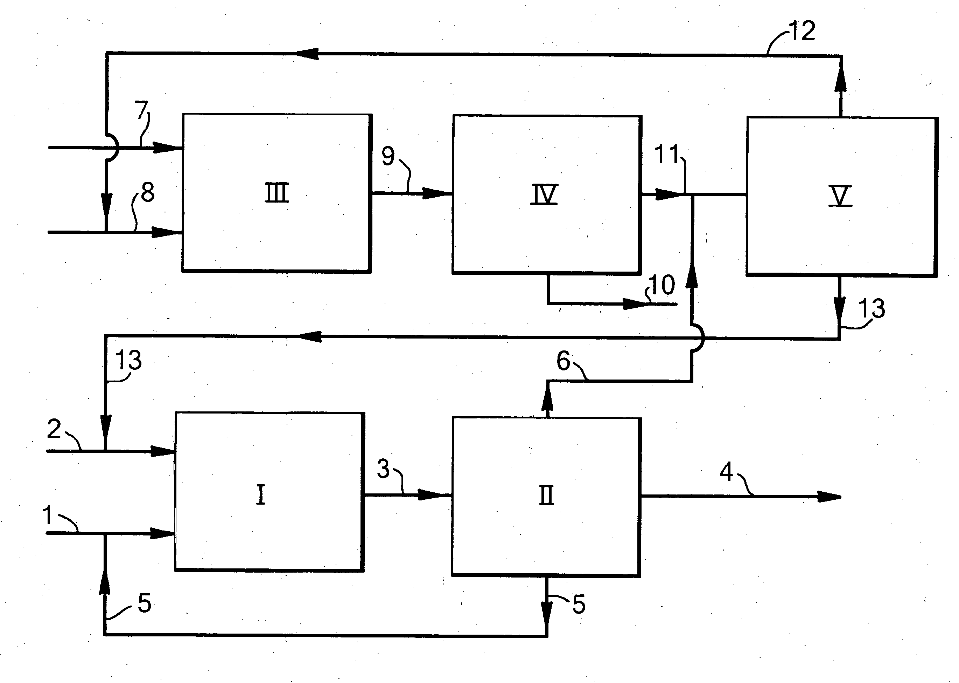

The figure depicts a flow scheme for the process according to the present invention.

As indicated above, the preparation of diaryl carbonates from the reaction of aromatic alcohol with dialkyl carbonate is known from, e.g., US-A 5,747,609. The reaction is generally performed in the presence of a catalyst. As indicated in the above-mentioned US document there are many types of catalysts, including lead compounds, salts or complexes of copper, silver or gold, alkali metal complexes, zinc complexes, cadmium complexes, iron, nickel or cobalt complexes, zirconium complexes, organotin compounds, Lewis acids and Lewis-acid-forming transition metal compounds, such as AlX₃, TiX₃, TiX₄, VOX₃, VX₅, ZnX₂, FeX₃ and SnX₄, in which X represents a halogen atom, an acetoxy group, an alkoxy group or an aryloxy group. Preferably, the catalyst is selected from the group consisting of compounds of lead, titanium, tin, and iron, which compounds contain halide, alkoxy or aryloxy moieties. More preferably, the alkoxy groups contain from 1 to 8 carbon atoms and the aryloxy group is phenoxy. Most preferably, the catalyst is a tetra-alkoxy titanium catalyst, wherein the alkoxy group contains from 2 to 6 carbon atoms, such as propoxy, butoxy, pentoxy and hexoxy groups.

In general the amounts of catalysts may vary between 0.001 to 2%wt, based on the total weight of dialkyl carbonate, aromatic hydroxyl compound, alkyl aryl carbonate, diaryl carbonate and alkyl alcohol. Preferred amounts include 0.005 to 1%wt, more preferred amounts being from 0.01 to 0.5%wt.

The reaction conditions have also been described in US-A 5,747,609. The residence time or reaction time is generally in the range of 0.001 to 50 hours, preferably from 0.01 to 10 hours, more preferably from 0.05 to 5 hours. The reaction temperature may be varied depending on the dialkyl carbonate used and diaryl carbonate produced. In general, the reaction temperature may range from 50 to 350°C, preferably from 120 to 280°C. The reaction pressure is not critical either and can be selected within wide ranges. It is feasible to conduct the reaction at sub-atmospheric, atmospheric and superatmospheric pressure. The reaction pressure is generally from 0.01 to 100 bar (1 kPa to 10 MPa), preferably from 1 to 50 bar.

The first transesterification may be conducted in a reactive distillation zone. In such a zone the separation of the first product stream containing diaryl carbonate, alkanol, unconverted dialkyl carbonate and unconverted aromatic alcohol already takes place in the transesterification zone. Unconverted dialkyl carbonate and alkanol, optionally together with entrained aromatic alcohol, are passed upwards in the zone and diaryl carbonate and aromatic alcohol are passed downwards in the zone. This is also shown in JP-A 09040616. The unconverted dialkyl carbonate and alkanol are withdrawn from the transesterification zone and are used as at least part of the second recycle stream. Alternatively, it is possible to conduct the first transesterification in a full liquid reactor such that the first product stream contains diaryl carbonate, alkanol, unconverted dialkyl carbonate and unconverted aromatic alcohol.

The first transesterification zone may be conducted in one reaction zone. However, it is preferred to employ at least two reaction zones. In the first zone the transesterification of dialkyl carbonate with one molecule of aromatic alcohol to alkyl aryl carbonate takes place. Subsequently, it is possible to make the alkyl aryl carbonate react with additional aromatic alcohol to yield diaryl carbonate and alkanol. Preferably, however, in the second zone the disproportionation reaction of two molecules of alkyl aryl carbonate to diaryl carbonate and dialkyl carbonate takes place. These reactions have been disclosed in, e.g., US-A 5,747,609, US-A 5,344,954 and US-A 5,210,268. Suitable reaction conditions include those disclosed in these documents, in particular in US-A 5,210,268. According to this document the residence time of the reactants in the liquid phase is in the range of 0.001 to 50 hr, preferably in the range from 0.01 to 10 hr, more preferably in the range from 0.05 to 2 hr. Suitably, the reaction conditions comprise a pressure of below 0.01 bar to above 200 bar, preferably from 0.05 to 50 bar, and a temperature of 50 to 350, preferably of 100 to 280°C. Catalysts, if present, may be selected from those that are also being used in the transesterification. Preferred catalyst are lead phenoxide (Pb(PhO)₂) or titanium phenoxide (Ti(PhO)₄) and dibutyl tin oxide (Bu₂SnO). However, also other catalysts, such as those disclosed in US-A 5,210,268 may be used.

In one or more subsequent separation zones, e.g. distillation zones, the separation between the diaryl carbonate-rich product stream, the aromatic alcohol-rich recycle stream and the second recycle stream comprising alkanol, dialkyl carbonate and aromatic alcohol is effected. Suitably this separation is effected in two distillation zones wherein in the first zone the separation between the second recycle stream and a mixture of aromatic alcohol and diaryl carbonate is obtained, followed by a separation between the aromatic alcohol and the diaryl carbonate. The aromatic alcohol-rich recycle stream is suitably recycled to the first transesterification zone. The diaryl carbonate-rich stream may be further purified as desired, e.g. by further distillation steps.

The conducting of the second transesterification, i.e. the conversion of alkylene carbonate with alkanol, is also known in the art. Reference is made to US-A 5,359,118 disclosing a process in which di(C₁-C₄ alkyl) carbonates are prepared by transesterification of an alkylene carbonate with a straight or branched C₁-C₄ alkanol.

The transesterification step is advantageously carried out in a column into which the alkylene carbonate is fed at the upper part, such that the alkylene carbonate flows down in counter current contact with upwardly moving alkanol. The product of the reaction is a dialkyl carbonate and an alkanediol. The dialkyl carbonate is recovered at the upper part of the column as the top stream. The alkanediol is recovered as the bottom stream. Alternatively, the transesterification step may be conducted in a conventional reactor, as disclosed in US-A 4,691,041.

The transesterification is suitably conducted in the presence of a catalyst. Suitable catalysts have been described in US-A 5,359,118 and include hydrides, oxides, hydroxides, alcoholates, amides, or salts of alkali metals, i.e., lithium, sodium, potassium, rubidium and caesium. Preferred catalysts are hydroxides or alcoholates of potassium or sodium. It is advantageous to use the alcoholate of the alkanol that is being used as feedstock. Such alcoholate can be added as such or being formed in situ.

Other suitable catalysts are alkali metal salts, such as alkali metal acetates, propionates, butyrates, or carbonates. Further suitable catalysts are described in US-A 5,359,118 and the references mentioned therein, such as EP-A 274 953, US-A 3,803,201, EP-A 1082, and EP-A 180 387.

Further suitable catalysts are heterogeneous catalysts, such as ion exchange resins that have been functionalised with tertiary amine groups, quaternary ammonium groups, sulphonic acid groups or carboxylic acid groups. Other heterogeneous catalysts include alkali metal and alkaline earth metal silicates on silica or ammonium-exchanged zeolites. These catalysts have been disclosed in US-A 4,691,041.

The transesterification conditions for the manufacture of dialkyl carbonates are known in the art and suitably include a temperature from 40 to 200°C, and a pressure from 0.05 to 40 MPa. Preferably, the pressure is from 0.1 to 1 MPa. The temperature depends on the alkanol feedstock and pressure used. When a reactive distillation zone is employed, the temperature is preferably kept such that it is close to and above the boiling point of the alkanol, e.g. up to 5°C above the boiling point. In the case of methanol and atmospheric pressure, the temperature is close to and above 65°C, for instance between 65 and 70°C.

When the second transesterification is conducted in a reactive distillation zone the separation of the alkanediol from the second product stream comprising the alkanediol, unconverted allcanol and dialkyl carbonate already takes place in the transesterification zone. Unconverted alkylene carbonate and alkanediol are passed downwards, and dialkyl carbonate and unconverted alkanol are passed upwards in the zone, as shown in US-A 5,359,118:

However, when the transesterification is conducted in a conventional reactor, as described in US-A 4,691,041, the separation of the second product stream is effected in separate separation zones, such as distillation zones. The distillation conditions may vary widely, as indicated in US-A 4,691,041. The pressures may vary from subatmospheric, e.g., 0.1 bar, to superatmospheric, e.g., 100 bar. Preferred distillation pressures range from 0.2 to 20 bar. The distillation temperatures are dependent on the pressure and the compounds used. Generally they range from 40 to 400°C.

When the alkanediol has been separated and recovered as product the remaining mixture of dialkyl carbonate and unconverted alkanol is to be distilled. The mixture is fed to the same distillation zone as the second recycle stream from the diaryl carbonate production, which second recycle stream comprises alkanol, dialkyl carbonate and aromatic alcohol. It is evident that this distillation can also handle any other contaminant that has been entrained with any of the streams, such as any unconverted alkylene carbonate that may be present in the mixture of dialkyl carbonate and unconverted alkanol. This distillation is conducted such that no aromatic alcohol is entrained in the alkanol stream that is withdrawn from the distillation zone as the low-boiling fraction. This entails that the dialkyl carbonate and the aromatic alcohol are recovered as the high-boiling products at the bottom of the distillation zone. The alkanol stream obtained in this step preferably does not contain significant amounts of dialkyl carbonate. Hence, the well-known azeotrope of methanol with a dialkyl carbonate, in particular dimethyl carbonate, is preferably avoided as the alkanol stream. The alkanol stream preferably contains from 0 to 10 %wt of dialkyl carbonate. When the stream contains more dialkyl carbonate the space time yield in the second transesterification zone is affected now that the dialkyl carbonate product is recycled to this zone.

The aromatic alcohol, contained in the contaminated stream containing dialkyl carbonate is recycled to the first transesterification zone. Since both the dialkyl carbonate and the aromatic alcohol are reactants in this zone, the fact that the stream is contaminated with aromatic alcohol does not affect the reaction.

The present invention can suitably be used for the preparation of alkanediols containing 2 to 6 carbon atoms. Thereto the alkylene carbonate is preferably obtained from the reaction of an oxirane with 2 to 6 carbon atoms with carbon dioxide. More preferably, the alkylene carbonate is ethylene carbonate or propylene carbonate. The alkanol in the second transesterification is suitably an alkanol with 1 to 4 carbon atoms. The alkanol may be straight or branched. The technology has been developed furthest using methanol. However, since methanol tends to form azeotropes with dialkyl carbonates, such as dimethyl carbonate, the use of methanol is not preferred. Preferably, the alkanol is ethanol, or a propanol, e.g. isopropanol. The aromatic alcohol that is used in the present invention is preferably phenol or phenol substituted with one or more alkyl groups, such as C₁-C₄ alkyl groups, suitably with a methyl group. Most preferably the aromatic alcohol is phenol.

The diaryl carbonate produced in the process of the present invention-is suitably used in the preparation of polycarbonates by the polymerisation with a dihydroxy aromatic compound, preferably with Bisphenol A. The invention thus also relates to use of the diaryl carbonate prepared in accordance with the above-described process, for the preparation of polycarbonates by the polymerisation with a dihydroxy aromatic compound, preferably with Bisphenol A. More in particular, the invention also relates to a process for the preparation of polycarbonates comprising preparing a diaryl carbonate by reaction of an aromatic alcohol with a dialkyl carbonate by the above-described process, and polymerising the diaryl carbonate with a dihydroxy aromatic compound, preferably with Bisphenol A.

The Figure shows a flow scheme of the process according to the present invention. An aromatic alcohol is passed into a first transesterification zone I via a line 1. Via a line 2 a dialkyl carbonate is also passed into transesterification zone I. A transesterification catalyst may be present in heterogeneous form in transesterification zone I or a homogeneous catalyst may be fed into the transesterification zone I with one of the reactants or via a separate line (not shown). As describe above the transesterification zone I may comprise several reactors to finally arrive at a product stream 3 that comprises diaryl carbonate, alkanol, unconverted dialkyl carbonate and unconverted aromatic alcohol. This product stream is fed into a separation zone II wherefrom a diaryl carbonate-rich product stream is discharged via a line 4, an aromatic alcohol-rich recycle stream is withdrawn via a line 5 and a second recycle stream comprising alkanol, dialkyl carbonate and aromatic alcohol is withdrawn via a line 6. The aromatic alcohol-rich recycle stream is recycled to the transesterification zone I via admixture with the aromatic alcohol feedstock in line 1.

In a second transesterification zone III an alkylene carbonate, introduced via a line 7, and an alkanol, introduced via a line 8 are reacted in the presence of a catalyst to yield a second product stream comprising alkanediol and unconverted alkanol and dialkyl carbonate. The catalyst may be present as a heterogeneous catalyst or fed as a homogeneous catalyst into the transesterification zone III with one of the reactants or via a separate line (not shown). The second product stream leave the zone III via a line 9 and is passed to a Second separation zone IV where in one or more distillation steps the second product stream is separated into an alkanediol product stream withdrawn via a line 10 and a mixture of dialkyl carbonate and unconverted alkanol discharged via a line 11. The mixture dialkyl carbonate and unconverted alkanol is mixed with the second recycle stream comprising alkanol, dialkyl carbonate and aromatic alcohol from line 6, and the resulting admixture is passed to a distillation zone V. Although the present embodiment describes the admixing of the stream in lines 11 and 6, the skilled person will understand that separate introduction of the two streams into the distillation zone V is also possible. In the distillation zone V separation is effected between an alkanol stream as the lower-boiling fraction and a contaminated stream comprising dialkyl carbonate and aromatic alcohol as the higher-boiling fraction. The alkanol stream is withdrawn via a line 12 and combined with the alkanol feedstock in line 8. It may be advantageous to fed the alkanol stream into transesterification zone III at a location separate from the location where line 8-debouches into the zone III. The contaminated stream comprising dialkyl carbonate and aromatic alcohol is passed via a line 13 to one of the reactants of the first transesterification zone I. In the drawing the admixing with the dialkyl carbonate stream in line 2 is shown. It is also possible to feed this contaminated stream separately into the zone I or to combine the stream with the aromatic alcohol stream in line 1.

In the present process the amounts of dialkyl carbonate and alkanol that have to be provided via lines 2 and 8, respectively, are minimal since the process provides a complete recycle of these products that are being generated in the process.

## Claims

1. Process for the preparation of diaryl carbonate by reaction of an aromatic alcohol with a dialkyl carbonate, which dialkyl carbonate has been prepared by the reaction of an alkanol and an alkylene carbonate, which process comprises the following steps:
(a) passing an aromatic alcohol and a dialkyl carbonate into a first transesterification zone to obtain a first product stream containing diaryl carbonate, alkanol, unconverted dialkyl carbonate and unconverted aromatic alcohol;
(b) separating the first product stream into a diaryl carbonate-rich product stream, an aromatic alcohol-rich recycle stream and a second recycle stream comprising alkanol, dialkyl carbonate and aromatic alcohol;
(c) feeding alkanol and alkylene carbonate into a second transesterification zone to obtain a second product stream comprising alkanediol and unconverted alkanol and dialkyl carbonate;
(d) separating alkanediol from the second product stream to yield an alkanediol product stream and a mixture of dialkyl carbonate and unconverted alkanol;
(e) subjecting the mixture of dialkyl carbonate and unconverted alkanol and the second recycle stream comprising alkanol, dialkyl carbonate and aromatic alcohol to the same distillation to obtain an alkanol stream as the lower-boiling fraction and a contaminated stream comprising dialkyl carbonate and aromatic alcohol as the higher-boiling fraction;
(f) recycling the alkanol stream of step e) to the second transesterification zone; and
(g) passing the contaminated stream comprising dialkyl carbonate and aromatic alcohol, and the aromatic alcoholrich recycle stream to the first transesterification zone.

2. Process according to claim 1, wherein the alkanol stream of step (e) contains from 0 to 10 %wt of dialkyl carbonate.

3. Process according to claim 1 or 2, wherein the aromatic alcohol is phenol.

4. Process according to any one of claims 1 to 3, wherein the alkylene carbonate is ethylene carbonate or propylene carbonate.

5. Process according to any one of claims 1 to 4, wherein the alkanol is a straight chain or branched C₁-C₄ alkanol.

6. Process for the preparation of polycarbonates comprising preparing a diaryl carbonate by reaction of an aromatic alcohol with a dialkyl carbonate by the process according to any one of claims 1 to 5, and polymerising the diaryl carbonate with a dihydroxy aromatic compound, preferably with Bisphenol A.

## Patentansprüche

1. Verfahren zur Herstellung von Diarylcarbonat durch Umsetzung eines aromatischen Alkohols mit einem Dialkylcarbonat, welches Dialkylcarbonat durch die Reaktion eines Alkanols und eines Alkylencarbonats hergestellt wird, welches Verfahren die folgenden Schritte umfasst:
(a) Leiten eines aromatischen Alkohols und eines Dialkylcarbonats in eine erste Umesterungszone, um einen ersten Produktstrom zu erhalten, enthaltend Diarylcarbonat, Alkanol, nicht umgesetztes Dialkylcarbonat und nicht umgesetzten aromatischen Alkohol;
(b) Auftrennen des ersten Produktstroms in einen an Diarylcarbonat reichen Produktstrom, einen an aromatischem Alkohol reichen Rezyklierungsstrom und einen zweiten Rezyklierungsstrom, umfassend Alkanol, Dialkylcarbonat und aromatischen Alkohol;
(c) Zuführen von Alkanol und Alkylencarbonat in eine zweite Umesterungszone, um einen zweiten Produktstrom zu erhalten, umfassend Alkandiol und nicht umgesetzten Alkanol und Dialkylcarbonat;
(d) Abtrennen des Alkandiols aus dem zweiten Produktstrom, um einen Alkandiolproduktstrom und eine Mischung aus Dialkylcarbonat und nicht umgesetztem Alkanol zu erhalten;
(e) Unterwerfen der Mischung aus Dialkylcarbonat und nicht umgesetztem Alkanol und dem zweiten Rezyklierungsstrom, umfassend Alkanol, Dialkylcarbonat und aromatischen Alkohol, unter die gleiche Destillation, um einen Alkanolstrom als die niedriger siedende Fraktion und einen kontaminierten Strom, umfassend Dialkylcarbonat und aromatischen Alkohol, als die höher siedende Fraktion zu erhalten;
(f) Rezyklieren des Alkanolstroms aus Schritt (e) zur zweiten Umesterungszone; und
(g) Leiten des kontaminierten Stroms, umfassend Dialkylcarbonat und aromatischen Alkohol, und des an aromatischem Alkohol reichen Rezyklierungsstroms zur ersten Umesterungszone.

2. Verfahren nach Anspruch 1, wobei der Alkanolstrom von Schritt (e) 0 bis 10 Gew-% Dialkylcarbonat enthält.

3. Verfahren nach Anspruch 1 oder 2, wobei der aromatische Alkohol Phenol ist.

4. Verfahren nach einem der Ansprüche 1 bis 3, wobei das Alkylencarbonat Ethylencarbonat oder Propylencarbonat ist.

5. Verfahren nach einem der Ansprüche 1 bis 4, wobei der Alkanol ein geradkettiger oder verzweigter C₁-C₄-Alkanol ist.

6. Verfahren zur Herstellung von Polycarbonaten, umfassend das Herstellen eines Diarylcarbonats durch Umsetzten eines aromatischen Alkohols mit einem Dialkylcarbonat durch das Verfahren nach einem der Ansprüche 1 bis 5, und Polymerisieren des Diarylcarbonats mit einer aromatischen Dihydroxyverbindung, vorzugsweise mit Bisphenol A.

## Revendications

1. Procédé de préparation de carbonate de diaryle par réaction d'un alcool aromatique avec un carbonate de dialkyle, ledit carbonate de dialkyle ayant été préparé par la réaction d'un alcanol et d'un carbonate d'alkylène, ledit procédé comprenant les étapes suivantes :
(a) le passage d'un alcool aromatique et d'un carbonate de dialkyle dans une première zone de transestérification pour obtenir un premier courant de produits contenant le carbonate de diaryle, l'alcanol, le carbonate de dialkyle non transformé et l'alcool aromatique non transformé ;
(b) la séparation du premier courant de produits en un courant de produits riche en carbonate de diaryle, un courant de recyclage riche en alcool aromatique et un second courant de recyclage comprenant l'alcanol, le carbonate de dialkyle et l'alcool aromatique ;
(c) l'introduction de l'alcanol et du carbonate d'alkylène dans une seconde zone de transestérification pour obtenir un second courant de produits comprenant un alcanediol et l'alcanol non transformé et le carbonate de dialkyle ;
(d) la séparation de l'alcanediol du second courant de produits pour obtenir un courant de produits alcanediol et un mélange de carbonate de dialkyle et d'alcanol non transformé ;
(e) la soumission du mélange de carbonate de dialkyle et d'alcanol non transformé et du second courant de recyclage comprenant l'alcanol, le carbonate de dialkyle et l'alcool aromatique à la même distillation pour obtenir un courant d'alcanol en tant que fraction de point d'ébullition inférieur et un courant contaminé comprenant le carbonate de dialkyle et l'alcool aromatique en tant que fraction de point d'ébullition supérieur ;
(f) le recyclage du courant d'alcanol de l'étape e) dans la seconde zone de transestérification ; et
(g) le passage du courant contaminé comprenant le carbonate de dialkyle et l'alcool aromatique et du courant de recyclage riche en alcool aromatique dans la première zone de transestérification.

2. Procédé selon la revendication 1, dans lequel le courant d'alcanol de l'étape (e) contient de 0 à 10 % en poids de carbonate de dialkyle.

3. Procédé selon la revendication 1 ou 2, dans lequel l'alcool aromatique est un phénol.

4. Procédé selon l'une quelconque des revendications 1 à 3, dans lequel le carbonate d'alkylène est le carbonate d'éthylène ou le carbonate de propylène.

5. Procédé selon l'une quelconque des revendications 1 à 4, dans lequel l'alcanol est un alcanol en C₁-C₄ linéaire ou ramifié.

6. Procédé de préparation de polycarbonates comprenant la préparation d'un carbonate de diaryle par réaction d'un alcool aromatique avec un carbonate de dialkyle par le procédé selon l'une quelconque des revendications 1 à 5, et polymérisation du carbonate de diaryle avec un composé dihydroxy aromatique, de préférence avec le bisphénol A.
